(19) Europäisches Patentamt
European Patent Office
Office européen des brevets

(11) Veröffentlichungsnummer: **0 039 814**
**B1**

(12) # EUROPÄISCHE PATENTSCHRIFT

(45) Veröffentlichungstag der Patentschrift: **21.09.83**

(51) Int. Cl.³: **C 07 D 409/04,** A 61 K 31/415 // C07D333/78

(21) Anmeldenummer: **81103132.7**

(22) Anmeldetag: **27.04.81**

(54) **Imidazolyl-indeno-thiophene, Verfahren zu ihrer Herstellung sowie ihre Verwendung als Arzneimittel.**

(30) Priorität: **09.05.80 DE 3017881**

(43) Veröffentlichungstag der Anmeldung: **18.11.81 Patentblatt 81/46**

(45) Bekanntmachung des Hinweises auf die Patenterteilung: **21.09.83 Patentblatt 83/38**

(84) Benannte Vertragsstaaten: **AT BE CH DE FR GB IT LI NL SE**

(56) Entgegenhaltungen:
**DE-A-1 811 654**
**FR-M-6 766**

(73) Patentinhaber: **BAYER AG, Zentralbereich Patente, Marken und Lizenzen, D-5090 Leverkusen 1, Bayerwerk (DE)**

(72) Erfinder: **Böshagen, Horst, Dr., Wiesenstrasse 4, D-5657 Haan (DE)**
Erfinder: **Büchel, Karl Heinz, Prof. Dr., Haus an der Dachsdelle Dabringhausener Strasse 42, D-5093 Burscheid (DE)**
Erfinder: **Draber, Wilfried, Dr., In den Birken 81, D-5600 Wuppertal 1 (DE)**
Erfinder: **Haller, Ingo, Dr., Viktoriastrasse 99, D-5600 Wuppertal 1 (DE)**
Erfinder: **Plempel, Manfred, Dr., Pahlkestrasse 5, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Imidazolyl-indeno-thiophene, Verfahren zu ihrer Herstellung sowie ihre Verwendung als Arzneimittel

Die Erfindung betrifft neue Imidazolyl-indeno-thiophene, ein Verfahren zu ihrer Herstellung sowie ihre Verwendung als Arzneimittel, insbesondere als Antimykotika.

Es ist bereits bekannt geworden, dass Imidazolyl-fluoren-Derivate, wie insbesondere das 9-(Imidazol-1-yl)-9-(2-methylphenyl)-fluoren, allgemein gute antimykotische Eigenschaften aufweisen (vergleiche DE-AS 18 11 654). Deren Wirkung ist jedoch, insbesondere gegen Dermatophyten, nicht immer ganz befriedigend.

Es wurden neue Imidazolyl-indeno-thiophene der allgemeinen Formel I

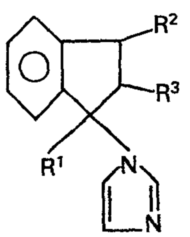

(I)

in welcher

$R^1$ für gegebenenfalls einfach oder mehrfach, gleich oder verschieden durch Alkyl mit 1–4 C-Atomen, Alkoxy oder Alkylthio, jeweils mit 1–2 C-Atomen, Halogen oder Halogenalkyl mit 1–2 C-Atomen mit 1–5 gleichen oder verschiedenen Halogen-, insbesondere Fluor oder Chloratomen, substituiertes Phenyl steht, und

$R^2$ und $R^3$ gemeinsam mit den C-Atomen, an die sie gebunden sind, für einen Thiophenrest stehen,

und deren physiologisch verträglichen Säureadditionssalze gefunden.

Weiterhin wurde gefunden, dass man die neuen Imidazolyl-indeno-thiophene der allgemeinen Formel I erhält, wenn man Halogen-indeno-thiophene der allgemeinen Formel II

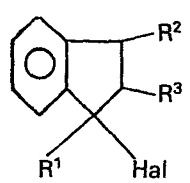

(II)

in welcher

$R^1$, $R^2$ und $R^3$ die oben angegebene Bedeutung haben und

Hal für Chlor oder Brom steht, mit Imidazol in Gegenwart eines Säurebindemittels und in Gegenwart eines Verdünnungsmittels umsetzt.

Die neuen Imidazolyl-indeno-thiophene weisen starke antimykotische Eigenschaften auf. Überraschenderweise zeigen sie eine bessere, therapeutisch nutzbare Wirksamkeit, insbesondere auch bei der lokalen Therapie bei Dermatophyten, als die aus dem Stand der Technik bekannten Imidazolyl-fluoren-Derivate, wie das 9-(Imidazol-1-yl)-9-(3-methylphenyl)-fluoren, welche chemisch und wirkungsmässig naheliegende Verbindungen sind. Die erfindungsgemässen Stoffe stellen somit eine Bereicherung der Pharmazie dar.

Die erfindungsgemässen Imidazolyl-indeno-thiophene sind durch die allgemeine Formel I allgemein definiert. In dieser Formel steht $R^1$ für gegebenenfalls einfach oder mehrfach, gleich oder verschieden substituiertes Phenyl, wobei als Substituenten vorzugsweise infrage kommen: Alkyl mit 1 bis 4 Kohlenstoffatomen, Alkoxy und Alkylthio mit jeweils 1 bis 2 Kohlenstoffatomen, Halogen sowie Halogenalkyl mit 1 bis 2 Kohlenstoff- und 1 bis 5 gleichen oder verschiedenen Halogenatomen, wie insbesondere Fluor- und Chloratomen.

Besonders bevorzugt sind diejenigen Verbindungen der allgemeinen Formel I, in denen $R^1$ für gegebenenfalls einfach oder zweifach, gleich oder verschieden substituiertes Phenyl steht, wobei als Substituenten infrage kommen: Fluor, Chlor, Brom, Methyl, Ethyl, Isopropyl, Methoxy, Methylthio und Trifluormethyl.

Im einzelnen seien ausser den bei den Herstellungsbeispielen genannten Verbindungen die folgenden Verbindungen der allgemeinen Formel I genannt:

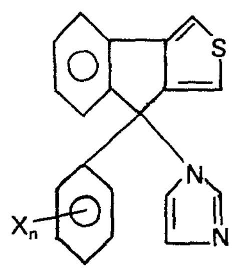

(Ia)

und

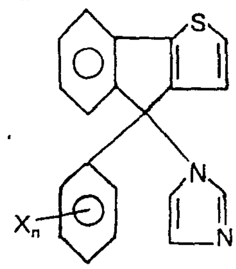

(Ib)

sowie

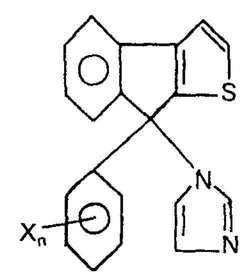

(Ic)

| $X_n$ | $X_n$ |
|---|---|
| – | 4-$SCH_3$ |
| 4-F | 3-$CF_3$ |
| 4-Cl | 2,3-$(CH_3)_2$ |
| 4-Br | 2,5-$(CH_3)_2$ |
| {3-Cl / 2-Cl} | 2,4-$(CH_3)_2$ |

Verwendet man beispielsweise 4-Chlor-4-(2-methylphenyl)-4H-indeno [1,2b]thiophen und Imidazol als Ausgangsstoffe, so kann der Reaktionsablauf durch das folgende Formelschema wiedergegeben werden:

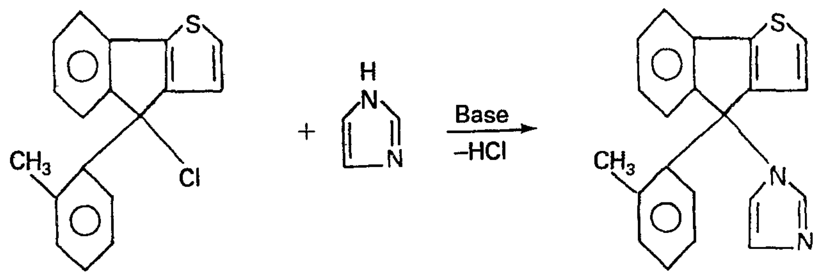


Die Halogen-indeno-thiophene der allgemeinen Formel II sind noch nicht bekannt. Sie lassen sich jedoch in allgemein bekannter Weise erhalten, indem man entsprechende Hydroxy-indeno-thiophene der allgemeinen Formel III

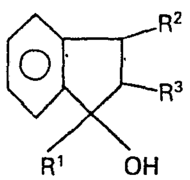

(III)

in welcher

$R^1$, $R^2$ und $R^3$ die oben angegebene Bedeutung haben, mit einem üblichen Halogenierungsmittel, wie z.B. Thionylchlorid, Thionylbromid, Phosphorylchlorid oder Phosphorylbromid, in Gegenwart eines inerten organischen Lösungsmittels, wie z.B. Benzol oder Toluol, bei Temperaturen zwischen 10 und 100 °C umsetzt. Die entstehenden Halogen-indeno-thiophene der allgemeinen Formel II können direkt weiter umgesetzt werden (vergleiche auch die Herstellungsbeispiele).

Die Hydroxy-indeno-thiophene der allgemeinen Formel III sind ebenfalls noch nicht bekannt. Sie werden erhalten, indem man bekannte Thiophenindenone (vergleiche z.B. J.Org.Chem. 35, 874 und J.Org.Chem. 32, 2441) in üblicher Weise mit einer metall-organischen Verbindung, wie insbesondere entsprechende Phenyl-lithium-Verbindungen, in Gegenwart eines inerten organischen Lösungsmittels, wie z.B. Diethylether oder Tetrahydrofuran, bei Temperaturen zwischen 0 und 30 °C umsetzt, und anschliessend in üblicher Weise hydrolisiert, wie insbesondere mit wässriger Schwefelsäure.

Als Verdünnungsmittel kommen für die erfindungsgemässe Umsetzung vorzugsweise polare organische Lösungsmittel infrage. Hierzu gehören vorzugsweise Nitrile, wie Acetonitril; Ketone, wie Aceton und Methylethylketon; Formamide, wie Dimethylformamid; sowie Nitromethan und Dimethylformamid; sowie Nitromethan und Dimethylsulfoxid. Die erfindungsgemässe Umsetzung wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie tertiäre Alkylamine, beispielsweise Triethylamin. Vorzugsweise verwendet man einen entsprechenden Überschuss an Imidazol.

Die Reaktionstemperaturen können bei der Durchführung des erfindungsgemässen Verfahrens in einem grösseren Bereich variiert werden. Im allgemeinen arbeitet man zwischen 20 und 150 °C, vorzugsweise bei 50 bis 100 °C.

Bei der Durchführung des erfindungsgemässen Verfahrens setzt man auf 1 Mol der Verbindung der allgemeinen Formel II 1 bis 2 Mol Imidazol und 1 bis 2 Mol Säurebinder ein. Die Isolierung der Endprodukte der allgemeinen Formel I erfolgt in allgemein üblicher Weise.

Zur Herstellung von physiologisch verträglichen Säureadditionssalzen der Verbindungen der allgemeinen Formel I kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen Verbindungen der allgemeinen Formel I und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sprosspilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabellen, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutische geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur Erfindung gehört auch die Verwendung der erfindungsgemässen Wirkstoffe sowie von pharmazeutischen Zubereitungen, die einen oder mehrere erfindungsgemässe Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

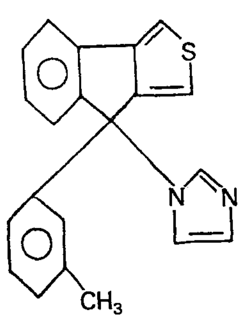

3,0 g (10 mMol) rohes 4-Chlor-4-(2-methyl-phenyl)-4H-indeno[1,2-c]thiophen werden in 15 ml Acetonitril gelöst, 2,1 g (30 mMol) Imidazol zugesetzt und die Mischung 5 Stunden unter Rückfluss erhitzt. Anschliessend wird im Vakuum eingeengt und der erhaltene sirupartige Rückstand mit 100 ml 2n Salzsäure versetzt. Die salzsaure Lösung wird mit Natronlauge alkalisch gestellt und ausgeethert. Aus dem getrockneten Ether-Extrakt erhält man 1,7 g Rohprodukt, das über Kieselgel chromatographiert wird (Laufmittel: Chloroform). Nach Umkristallisation aus Methanol erhält man 1,1 g (33,5% der Theorie) 4-(Imidazol-1-yl)-4-(2-methyl-phenyl)-4H-indeno[1,2-c]-thiophen vom Schmelzpunkt 176 °C.

Herstellung der Vorstufen

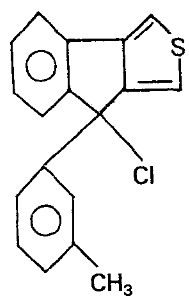

2,8 g (10 mMol) 4-Hydroxy-4-(2-methyl-phenyl)-4H-indeno-[1,2-c]thiophen werden in 20 ml Benzol gelöst und 18 ml Thionylchlorid zugetropft. Die Mischung wird 5 Stunden unter Rückfluss erhitzt, dann im Vakuum eingedampft. Der erhaltene Sirup wird nochmals in 20 ml Benzol aufgenommen und wieder im Vakuum eingedampft.

Man erhält 3,0 g rohes 4-Chlor-4-(2-methyl-phenyl-4H-indeno[1,2-c]thiophen als zähflüssiges Öl, das direkt weiter umgesetzt wird.

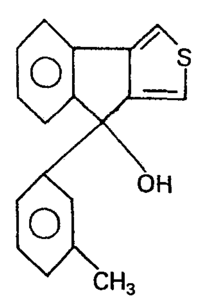

5,0 g (27 mMol) 4H-Indeno[1,2-c]thiophen-4-on in 50 ml Ether/Tetrahydrofuran (1:1) gelöst und in 200 ml einer etherischen Lösung von 50 mMol 2-Methyl-phenyllithium eingetropft. Die Reaktionsmischung wird 6 Stunden bei Raumtemperatur gerührt. Unter Eiskühlung werden anschliessend 85 ml 1 n Schwefelsäure zugetropft, die etherische Phase abgetrennt, getrocknet und eingedampft. Man erhält 13 g Rohprodukt, welches über eine Kieselgelsäure chromatographiert wird (Laufmittel: Chloroform). Man erhält 2,8 g (37,5% der Theorie) 4-Hydroxy-4-(2-methyl-phenyl)-4H-indeno[1,2-c]thiophen als gelbliches zähflüssiges Öl.

Beispiel 2

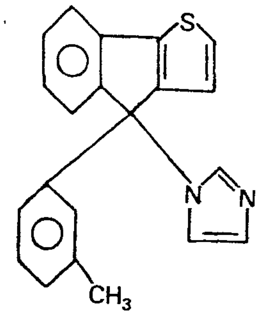

6,1 g (20 mMol) rohes 4-Chlor-4-(2-methyl-phenyl)-4H-indeno-[1,2-b]thiophen werden in 30 ml Acetonitril aufgenommen, 4,1 g (60 mMol) Imidazol zugesetzt und die Mischung 5 Stunden unter Rückfluss erhitzt. Anschliessend wird das Rohprodukt durch Zugabe von 70 ml Wasser als Sirup gefällt. Die überschüssige Lösung wird dekantiert und das Rohprodukt in Ether aufgenommen. Die Etherlösung wird getrocknet und eingedampft. Der Rückstand wird in 100 ml 2 n Salzsäure aufgenommen, vom Unlöslichen abgetrennt, nach Abkühlen mit Natronlauge die Base erneut freigesetzt und mit Ether extrahiert. Die organische Phase wird über Natriumsulfat getrocknet und eingeengt. Man erhält 4,1 g (62,1% der Theorie) 4-(Imidazol-1-yl)-4-(2-methyl-phenyl)-4H-indeno[1,2-b]-thiophen vom Schmelzpunkt 75 °C.

Herstellung der Vorstufen

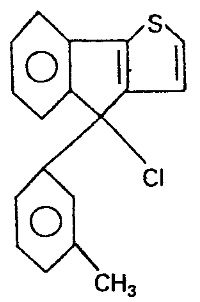

5,6 g (20 mMol) 4-Hydroxy-4-(2-methyl-phenyl)-4H-indeno-[1,2-b]-thiophen werden in 40 ml absolutem Benzol gelöst und 34 ml Thionylchlorid zugegeben. Die Mischung wird 5 Stunden unter Rückfluss erhitzt. Dann wird die Reaktionslösung im Vakuum eingedampft, der ölige Rückstand in Cyclohexan aufgenommen, Unlösliches abfiltriert und das klare Filtrat erneut im Vakuum eingedampft. Man erhält 6,1 g rohes 4-Chlor-4-(2-methyl-phenyl)-4H-indeno[1,2-b]thiophen als zähflüssiges Öl, das direkt weiter umgesetzt wird.

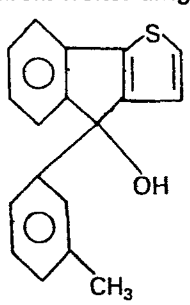

5,0 g (27 mMol) 4H-Indeno[1,2-b]thiophen-4-on in 50 ml Ether gelöst und bei Raumtemperatur 50 mMol 2-Methylphenyllithium in 250 ml absolutem Ether eingetropft. Die Reaktionslösung wird 6 Stunden bei Raumtemperatur nachgerührt und dann durch Zugabe von 100 ml 1 n Schwefelsäure zersetzt. Die Etherphase wird abgetrennt, über Natriumsulfat getrocknet und eingeengt.

Man erhält 12,3 g Rohprodukt, welches über eine Kieselgel-Säule chromatographiert wird (Laufmittel: Chloroform). Nach Umkristallisation aus Cyclohexan erhält man 4,9 g (65% der Theorie) 4-Hydroxy-4-(2-methyl-phenyl)-4H-indeno [-1,2-b]thiophen vom Schmelzpunkt 122 °C.

Beispiel 3

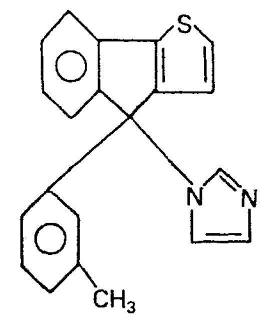

x ½

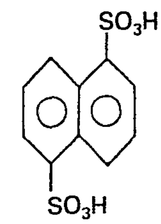

Durch Umsetzung von 4-(Imidazol-1-yl)-4-(2-methyl-phenyl)-4H-indeno-[1,2-b]thiophen (Beispiel 2) mit 1,5-Naphthalindisulfonsäure erhält man nahezu quantitativ 4-(Imidazol-1-yl)-(2-methyl-phenyl)- 4H-indeno[1,2-b]thiophen-naphthalindisulfonat-(1,5) vom Schmelzpunkt 243 °C.

Anwendungsbeispiele

In den nachfolgenden Beispielen werden die nachstehend angegebenen Verbindungen als Vergleichssubstanzen eingesetzt:

(A)

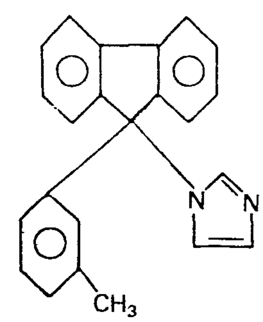

(B)

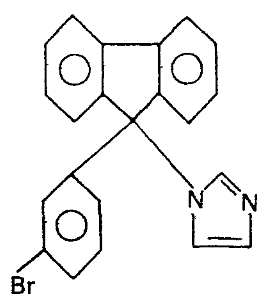

Beispiel A
Antimykotische in-vitro-Wirksamkeit
Versuchsbeschreibung:

Die in-vitro-Prüfung wurde im Reihenverdünnungstest mit Keiminokula von durchschnittlich 5×10 Keimen/ml Substrat durchgeführt. Als Nährmedium dienten

a) für Dermatophyten und Schimmelpilze: Sabourand's milieu d'épresive

b) für Hefen:
Isotonic Sensitest-Broth von Oxid.

Die Bebrütungstemperatur betrug 28 °C; Bebrütungszeit war 24 Stunden bei Hefen und 96 Stunden bei Dermatophyten und Schimmelpilzen.

In diesem Test zeigen die erfindungsgemässen Verbindungen 1 und 2 eine bessere Wir'kung als die aus dem Stand der Technik bekannten Verbindungen (A) und (B).

Beispiel B
Antimikrobielle in-vivo-Wirksamkeit (lokal) am Modell der experimentellen Meerschweinchen-Trichophytie

Versuchsbeschreibung:

Weisse Meerschweinchen der Rasse Pirbrightwhite wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p.i., 1× täglich mit 1%-iger Lösung der erfindungsgemässen Präparate (in Dimethylsulfoxid:Glycerin = 1:4) lokal behandelt.

Ergebnis:

Bei unbehandelten Tieren entwickelt sich innerhalb von 12 Tagen p.i. das typische Bild einer Dermatophytose mit Rötung, Schuppung und Haarausfall bis zum totalen Integument-Defekt an der Infektionsstelle.

Die erfindungsgemässe Verbindung 2 zeigt keine Infektionszeichen, während es bei den bekannten Verbindungen (A) und (B) zu Rötung, Schuppung und teilweise auch zu Haarausfall kommt.

**Patentansprüche**

1. Imidazolyl-indeno-thiophene der allgemeinen Formel I

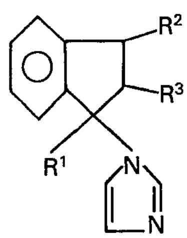

(I)

in welcher

$R^1$ für gegebenenfalls einfach oder mehrfach, gleich oder verschieden durch Alkyl mit 1–4 C-Atomen, Alkoxy oder Alkylthio, jeweils mit 1–2 C-Atomen, Halogen oder Halogenalkyl mit 1–2 C-Atomen mit 1–5 gleicher oder verschiedenen Halogenatomen, substituiertes Phenyl steht, und

$R^2$ und $R^3$ gemeinsam mit den C-Atomen, an die sie gebunden sind, für einen Thiophenrest stehen, und deren physiologisch verträglichen Säureadditionssalze.

2. Imidazolyl-indeno-thiophene der allgemeinen Formel I, in der $R^2$ und $R^3$ die oben angegebene Bedeutung haben und

$R^1$ für gegebenenfalls einfach oder mehrfach, gleich oder verschieden durch Fluor, Chlor, Brom, Methyl, Ethyl, Isopropyl, Methoxy, Methylthio oder Trifluormethyl substituiertes Phenyl steht, und deren physiologisch verträglichen Säureadditionssalze.

3. 4-(Imidazol-1-yl)-4-(2-methyl-phenyl)-4H-indeno-[1,2-c]thiophen.

4. 4-(Imidazol-1-yl)-4-(2-methyl-phenyl)-4H-indeno[1,2-b]thiophen.

5. 4-(Imidazol-1-yl)-4-(2-methyl-phenyl)-4H-indeno-[1,2-b]thiophen-naphthalindisulfonat-(1,5).

6. Verfahren zur Herstellung von Imidazolyl-indeno-thiophenen der allgemeinen Formel I, dadurch gekennzeichnet, dass man Halogen-indeno-thiophene der allgemeinen Formel II

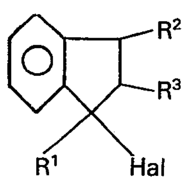

(II)

in welcher

$R^1$, $R^2$ und $R^3$ die oben angegebene Bedeutung haben und

Hal für Chlor oder Brom steht, mit Imidazol in Gegenwart eines Säurebindemittels und in Gegenwart eines Verdünnungsmittels umsetzt.

7. Arzneimittel, gekennzeichnet, durch einen Gehalt an mindestens einem Imidazolyl-indeno-thiophen gemäss Anspruch 1.

8. Verfahren zur Herstellung von antimykotischen Mitteln, dadurch gekennzeichnet, dass man Imidazolyl-indeno-thiophen gemäss Anspruch 1 mit inerten, nichttoxischen pharmazeutisch geeigneten Trägerstoffen vermischt.

**Revendications**

1. Imidazolyl-indéno-thiophènes de formule générale I:

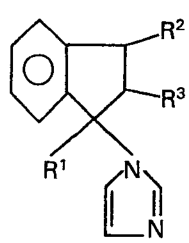

(I)

dans laquelle

$R^1$ représente un groupe phényle éventuellement substitué une ou plusieurs fois de manière identique ou différente par un groupe alkyle contenant 1 à 4 atomes de carbone, par un groupe alcoxy ou alkylthio contenant chacun 1 ou 2 atomes de carbone, par un atome d'halogène ou un groupe halogénalkyle contenant 1 ou 2 atomes

de carbone avec 1 à 5 atomes d'halogènes identiques ou différents, et

$R^2$ et $R^3$, ensemble avec les atomes de carbone auxquels ils sont reliés, représentent un groupe thiophène, ainsi que leurs sels d'addition d'acide physiologiquement compatibles.

2. Imidazolyl-indéno-thiophènes de formule générale I dans laquelle $R^2$ et $R^3$ ont les significations indiquées ci-dessus, et

$R^1$ représente un groupe phényle éventuellement substitué une ou plusieurs fois de manière identique ou différente par un atome de fluor, un atome de chlore, un atome de brome, un groupe méthyle, un groupe éthyle, un groupe isopropyle, un groupe méthoxy, un groupe méthylthio ou par un groupe trifluorométhyle, de même que leurs sels d'addition d'acide physiologiquement compatibles.

3. Le 4-(imidazol-1-yl)-4-(2-méthyl-phényl)-4H-indéno[1,2-c]thiophène.

4. Le 4-(imidazol-1-yl)-4-(2-méthyl-phényl)-4H-indéno[1,2-b]thiophène.

5. Le naphtalène- disulfonate- (1,5) du 4-(imdazol-1-yl)-4- (2-méthyl-phényl)- 4H-indéno[1,2-b] thiophène.

6. Procédé de préparation d'imidazolyl-indéno-thiophènes de formule générale I, caractérisé en ce qu'on fait réagir des halogéno-indéno-thiophènes de formule générale II:

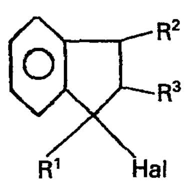

(II)

dans laquelle

$R^1$, $R^2$ et $R^3$ ont les significations indiquées ci-dessus et

Hal représente un atome de chlore ou de brome, avec l'imidazole en présence d'un agent fixateur d'acide et en présence d'un diluant.

7. Médicament caractérisé en ce qu'il contient au moins un imidazolyl-indéno-thiophène suivant la revendication 1.

8. Procédé de préparation d'agents antimycotiques, caractérisé en ce qu'on mélange un imidazolyl-indéno-thiophène suivant la revendication 1 avec des substances supports inertes, non toxiques et pharmaceutiquement appropriées.

**Claims**

1. Imidazolyl-indeno-thiophenes of the general formula I

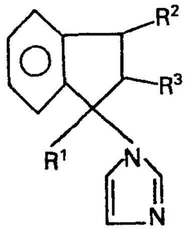

(I)

in which

$R^1$ represents phenyl which is optionally monosubstituted or polysubstituted by identical or different substituents from the group comprising alkyl with 1–4 C atoms, alkoxy or alkylthio, in each case with 1–2 C atoms, halogen or halogenoalkyl with 1–2 C atoms and with 1–5 identical or different halogen atoms, and

$R^2$ und $R^3$, together with the C atoms to which they are bonded, represent a thiophene radical, and physiologically acceptable acid addition salts thereof.

2. Imidazolyl-indeno-thiophenes of the general formula I, in which

$R^2$ and $R^3$ have the meaning indicated above and

$R^1$ represents phenyl which is optionally monosubstituted or polysubstituted by identical or different substituents from the group comprising fluorine, chlorine, bromine, methyl, ethyl, isopropyl, methoxy, methylthio or trifluoromethyl, and physiologically acceptable acid addition salts thereof.

3. 4-(Imidazol-1-yl)-4-(2-methyl-phenyl)-4H-indeno-[1,2-c]thiophene.

4. 4-(Imidazol-1-yl)-4-(2-methyl-phenyl)-4H-indeno[1,2-b]thiophene.

5. 4-(Imidazol-1-yl)-4-(2-methyl-phenyl)-4H-indeno[1,2-b]thiophene 1,5-naphthalenedisulphonate.

6. Process for the preparation of imidazolyl-indeno-thiophenes of the general formula I, characterised in that halogeno-indeno-thiophenes of the general formula II

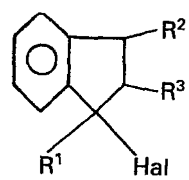

(II)

in which

$R^1$, $R^2$ and $R^3$ have the abovementioned meaning and

Hal represents chlorine or bromine, are reacted with imidazole in the presence of an acidbinding agent and in the presence of a diluent.

7. Medicaments, characterised in that they contain at least one imidazolyl-indeno-thiophene according to Claim 1.

8. Process for the preparation of antimycotic agents, characterised in that imidazolyl-indeno-thiophene according to Claim 1 is mixed with inert, non-toxic, pharmaceutically suitable excipients.